(19) Europäisches Patentamt
European Patent Office
Office européen des brevets

(11) EP 1 925 608 B1

(12) **EUROPEAN PATENT SPECIFICATION**

(45) Date of publication and mention of the grant of the patent:
**08.03.2017 Bulletin 2017/10**

(21) Application number: **06796580.6**

(22) Date of filing: **21.08.2006**

(51) Int Cl.:
***C07C 41/26*** *(2006.01)* ***C07C 43/13*** *(2006.01)*

(86) International application number:
**PCT/JP2006/316317**

(87) International publication number:
**WO 2007/023765 (01.03.2007 Gazette 2007/09)**

(54) **PROCESS FOR PRODUCING HYDROLYZATE**

VERFAHREN ZUR HERSTELLUNG EINES HYDROLYSATS

PROCÉDÉ DE PRODUCTION D'HYDROLYSAT

(84) Designated Contracting States:
**DE ES FR**

(30) Priority: **22.08.2005 JP 2005240276**

(43) Date of publication of application:
**28.05.2008 Bulletin 2008/22**

(73) Proprietor: **Kao Corporation**
**Chuo-ku**
**Tokyo 103-8210 (JP)**

(72) Inventor: **NAKA, Kenichi**
**Wakayama-shi**
**Wakayama 640-8580 (JP)**

(74) Representative: **Hoffmann Eitle**
**Patent- und Rechtsanwälte PartmbB**
**Arabellastraße 30**
**81925 München (DE)**

(56) References cited:
**JP-A- 2003 267 902**

- **PATENT ABSTRACTS OF JAPAN vol. 2003, & JP 2003 267902 A (KAO CORP), 25 September 2003 (2003-09-25)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 925 608 B1

## Description

TECHNICAL FIELD

The present invention relates to a method for producing a hydrolysate.

BACKGROUND ART

Hydrolysis reaction such as ring-opening reaction of an organic compound and the like is industrially used in many situations. For example, glyceryl ether obtained by ring-opening reaction of glycidyl ether is a compound useful as a solvent, an emulsifier, a dispersant, a detergent, a foam booster and the like.

Although glyceryl ether is produced using a catalyst in general, as a method for producing glyceryl ether without using a catalyst, for example, a method in which glycidyl ether is brought into a hydrolysis reaction with subcritical water or the like has been known (see Japanese Laid-Open Publication No. 2002-88000).

However, in the known method, depending on a mixture state of an organic compound and water, delay in reaction time, a side reaction of dimerizing the organic compound as a raw material and a generated hydrolysate are increased or like problems are caused.

JP 2003-267920 relates to a method of producing glyceryl ethers by means of a hydrolysis reaction. The temperature in the reaction system is controlled to fall within a predetermined range. Water and the material are supplied to a reactor without being mixed in advance. A mixer with high shear properties is preferably used.

DISCLOSURE OF THE INVENTION

The present invention provides a method for effectively producing a high quality hydrolysate by making a mixture state of an organic compound and water be a good condition for reaction.

To achieve this, the present invention is directed to a method for producing a hydrolysate in which an organic compound as defined below and water are mixed and a hydrolysis reaction of the organic compound is subsequently performed. In the method, the mixing is performed under shear flow of the organic compound and the water at a shear rate U/Dmin of 20 $sec^{-1}$ or more (where Dmin is a flow channel minimum inner diameter (mm) in a mixing section and U is a flow rate (mm/sec) of a mixture of the organic compound and the water in the mixing section), and the hydrolysis reaction of the organic compound is performed at a reaction temperature of 150 °C to 350 °C and a reaction pressure equal to or higher than a saturation vapor pressure of the water.

BRIEF DESCRIPTION OF THE DRAWINGS

[FIG. 1] FIG. 1 is a diagram schematically illustrating an exemplary device preferably used in an embodiment of a method for producing a hydrolysate according to the present invention.

[FIG. 2] FIG. 2 is a diagram schematically illustrating another exemplary device preferably used in an embodiment of a method for producing a hydrolysate according to the present invention.

BEST MODE FOR CARRYING OUT THE INVENTION

The present invention is characterized in that when an organic compound and water are mixed to perform a hydrolysis reaction of the organic compound, under predetermined conditions, the organic compound and water are mixed and hydrolysis of the organic compound is performed.

According to the present invention, decomposition and a side reaction of an organic compound as a raw material can be suppressed in the hydrolysis reaction and thus deterioration of hue of the generated hydrolysate and the like can be prevented. Furthermore, according to the present invention, since the hydrolysis reaction is performed under a high temperature condition, the reaction can proceed with high selectivity even without using a catalyst, the step of removing a catalyst from a reactant is not necessary, and a high quality hydrolysate can be efficiently produced.

As long as the components do not inhibit achievement of desired effects of the present invention, components and the like described below can be used independently or two or more of the components can be combined and then used.

The hydrolysis reaction is preferably a ring-opening reaction using water. The organic compound used as a raw material is a compound which has a ring structure and of which ring structure is opened due to the hydrolysis reaction. As such a compound, glycidyl ether expressed by the following general formula (I) is used.

[Chemical formula 1]

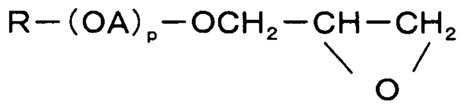

(I)

(where R is a hydrocarbon group in which part or all hydrogen atoms may be replaced with fluorine atoms, of which carbon number is 1 to 20 and which is saturated or unsaturated, OA is an oxyalkylene group which may be the same as or different from another OA and of which carbon number is 2 to 4, and p is a number of 0 through 20). Gryceryl ether obtained by ring-opening of glycidyl ether is a compound useful as a solvent, an emulsifier, a dispersant, a detergent, or a foam booster.

In the above formula, as the hydrocarbon group denoted by R, in which part or all hydrogen atoms may be replaced with fluorine atoms and of which carbon number is 1 to 20, for example, a straight-chain or branched-chain alkyl group of which carbon number is 1 to 20, a straight-chain or branched-chain alkenyl group of which carbon number is 2 to 20, an aryl group of which carbon number is 6 to 14 or the like may be used.

As the hydrocarbon group, specifically, a methyl group, an ethyl group, a n-propyl group, a n-butyl group, a n-pentyl group, a n-hexyl group, a n-heptyl group, a n-octyl group, a n-nonyl group, a n-decyl group, a n-dodecyl group, a tetradecyl group, a hexadecyl group, an octadecyl group, an eicosyl group, a 2-propyl group, a 2-butyl group, a 2-methyl-2-propyl group, a 2-pentyl group, a 3-pentyl group, a 2-hexyl group, a 3-hexyl group, a 2-octyl group, a 2-ethylhexyl group, a phenyl group, a benzyl group or the like can be used. Also, as the hydrocarbon group in which hydrogen atoms are replaced with fluorine atoms, for example, there are a perfluoroalkyl group such as a nanofluorohexyl group, a hexafluorohexyl group, a tridecafluorooctyl group, a heptadecafluorooctyl group, a heptadecafluorodecyl group and the like, obtained by replacing hydrogen atoms of the above-described hydrocarbon groups with fluorine atoms in an arbitrary manner without particular limits of the degree and location of replacement.

As specific examples of oxyalkylene group denoted by OA, of which carbon number is 2 to 4 are alkylene oxide such as an oxyethylene group, an oxytrimethylene group, an oxypropylene group, an oxybutylene group and the like.

Note that the carbon number of the hydrocarbon group denoted as R is preferably 1 to 12 in view of improving selectivity. Moreover, as for p, a number of 0 to 6 is preferable and 0 is more preferable.

According to the present invention, specifically, as glycidyl ether preferably used as a raw material, for example, n-butylglycidyl ether, 2-methyl-propylglycidyl ether, n-pentylglycidyl ether, 2-methy-butylglycidyl ether, n-hexylglycidyl ether, 2-methyl-pentylglycidyl ether, phenylglycidyl ether, n-octylglycidyl ether, 2-ethyl-hexylglycidyl ether, isodecylglycidyl ether, n-stearylglycidyl ether and the like can be used.

According to the present invention, types of water used for the hydrolysis reaction of a raw material are not limited. As such water, for example, ion-exchange water, distilled water, or reverse osmosis filtered water can be used. Within the range in which nature of the present invention is not impaired, use of water containing e.g. salt such as tap water is no problem.

An amount of water with respect to an organic compound is not particularly limited. However, in terms of molar conversion, it is preferably 20 to 500 times as large as a stoichiometric amount of water required for a reaction, more preferably 40 to 300 times as large as the stoichiometric amount thereof, and furthermore preferable 70 to 200 times as large as the stoichiometric amount thereof. In the above-described range, a side reaction such as dimerization of an organic compound as a raw material and a generated hydrolysate and the like can be suppressed and thus the selectivity of the hydrolysate can be further increased.

In the production method according to the present invention, the above-described organic compound as a raw material and water are mixed and then a hydrolysis reaction is performed.

According to the present invention, an organic compound and water are mixed in a mixer and then a hydrolysis reaction is performed in a reactor. In this aspect, the mixer corresponds to the mixing section and the reactor corresponds to the reaction section.

Mixing of an organic compound and water is performed at a shear rate (U/Dmin) of 20 ($sec^{-1}$) or more, and preferably at a shear rate (U/Dmin) of 100 ($sec^{-1}$) or more. By performing shear flow of an organic compound and water to mix the organic compound and water, the organic compound and water can be reacted in a good mixed state through a hydrolysis reaction. Accordingly, it is preferable that after the mixture of the organic compound and water, the mixture is quickly brought to a hydrolysis reaction while keeping the mixed state as it is.

For shear rate U/Dmin, Dmin is a flow channel minimum inner diameter (mm) and U is a flow rate (mm/sec) of the mixture of the organic compound and water at Dmin. Assuming that the flow rate of the mixture is Q (ml/sec), U (mm/sec) can be obtained based on the following formula (1) where n is the number of the flow channel minimum inner

diameter. For example, when a porous contraction flow type mixer is used, n is the number of pores in the mixer.

$$U\ (mm/sec) = Q \times 1000/(n \times \pi \times (Dmin)^2/4) \qquad (1)$$

Moreover, a cross-sectional shape of the mixing portions does not have to be a circular shape. When the cross-sectional shape is other than a circular shape, U is a flow rate at a flow channel minimum cross-sectional area. In that case, a diameter of a circle having the same area as the flow channel minimum cross section is used as Dmin.

The flow channel minimum inner diameter of the mixing section is preferably 1 mm or more in view of productivity and is preferable 15 mm or less in view of achieving a good mixed state of an organic compound and water. In consideration of these views, the flow channel minimum inner diameter is preferably 1 to 15 mm and more preferably 1 to 10 mm. The flow channel minimum inner diameter of the mixing section means the flow channel minimum inner diameter of the mixer.

A mixing time is not particularly limited as long as the mixing time is long enough to sufficiently mix an organic compound and water. In the case of a continuous type mixer, in general, the mixing time is preferably selected to be within the range from about 0.001 seconds to 10 hours. The range from about 0.001 seconds to 1 hour is more preferable and the range from 0.001 seconds to 10 minutes is further more preferable. The mixing time for a continuous type mixer means a time in which a reaction liquid is retained in the mixer and is indicated by a value obtained by dividing a volume of the mixer by a flow volume of reaction materials supplied to the reactor per unit time.

In view of increasing reactivity of an organic compound and water and in view of suppressing corrosion of a reactor, a reaction temperature for a hydrolysis reaction is 150 °C to 350 °C, preferably 200 °C to 300 °C and more preferably 250 °C to 290 °C. As for a reaction pressure, a hydrolysis reaction is performed under a condition where a pressure equal to or higher than a saturation vapor pressure of water is applied and water can be kept to be in a liquid state.

A reaction time varies depending on a reaction temperature, a type of a raw material to be used and the like and therefore can not be determined. However, in general, the reaction time is preferably selected to be within the range from 0.1 minute to 10 hours. The range from 0.1 minute to 1 hour is more preferable and the range from 0.1 minute to 10 minutes is further more preferable. In the case of a batch type reactor, the reaction time is counted from the completion of loading a raw material. In the case of a continuous type reactor, the reaction time is counted from a timing at which a reaction has reached a stationary state. The reaction time of the continuous type reactor means a time in which a reaction liquid is retained in the reactor and is indicated by a value obtained by dividing a volume of the reactor by a flow volume of reaction materials supplied to the reactor per unit time.

According to the present invention, a hydrolysis reaction is preformed at a high temperature. Thus, the reaction proceeds even without a catalyst. However, an acid or alkali catalyst can be added. A catalyst used in the present invention is not particularly limited but, for example, an acid, a base or a combination of an acid and a base, which are in general used in hydrolysis reaction, can be used.

When a catalyst is used, a usage amount of the catalyst is not particularly limited as long as a desired reaction efficiency of a hydrolysis reaction of a raw material is achieved. However, in general, the usage amount is preferably 0.01 to 10 parts by weight and more preferably 0.1 to 5 parts by weight with respect to 100 parts by weight of an organic compound as a raw material.

As a mixer used for mixing an organic compound and water in advance, in the case of a batch type mixer, for example, a propeller mixer, an agihomo-mixer, a homo-mixer, a disk turbine paddle impeller having a high shear property, a pitched blade paddle impeller, or a paddle blade impeller can be preferably used. In the case of a continuous type mixer, for example, a pipe line mixer, a line homo-mixer, an ultrasonic mixer, a high pressure homogenizer, pumps such as a centrifugal pump having a high shear property, a disper mixer, or a static mixer can be preferably used. Among the above-described examples, it is preferable to use a static mixer because it has a simple configuration and its maintenance can be done in a simple manner, compared to the other ones. Specifically, an orifice contraction flow type mixer is more preferable because a fluid shear rate at a mixing section thereof is high and high mixing effect can be achieved.

A temperature when an organic compound and water are mixed in a mixer is not particularly limited but is preferably about the same temperature as a reaction temperature.

As a reactor for performing a hydrolysis reaction, in the case of a continuous type reactor, a flow tube type reactor such as a tube type reactor, a tower type reactor, a semi-batch reactor such as a continuous stirred tank reactor can be used.

A material of the reactor used according to the present invention is not particularly limited. In general, a material used for chemical reaction can be arbitrarily used. Specific examples are metal materials such as steel, stainless steel, Fe-Cr-Ni alloy such as carpenter 20, copper alloy, aluminum alloy, Ni-Cr-Fe alloy, Ni-Cu alloy, Ni-Mo-Fe-Cr alloy, cobalt alloy, titanium alloy, zirconium alloy, molybdenum, or chromium, hard glass, silica glass, porcelain, glass lining, synthetic resin, or ceramic materials. Among the above-described materials, when a reaction takes place under a temperature condition close to a supercritical water condition where corrosion of materials is concerned, a metal material such as

austenitic stainless steel, Ni-Cr-Fe alloy, or Ni-Mo-Fe-Cr alloy is preferable and Ni-Cr-Fe alloy and Ni-Mo-Fe-Cr alloy are more preferable.

According to the method of the present invention, a hydrolysis reaction can be performed by either one of a batch method in which a raw material at a required amount for 1 batch is supplied and a hydrolysis reaction for the amount is completed in a batch operation and a continuous method in which a raw material is continuously supplied and a hydrolysis reaction is performed. However, because a temperature can be increased/reduced in a short time, reaction conditions can be controlled in a simple manner and a reaction can be made to effectively proceed, it is preferable to continuously perform a hydrolysis reaction.

When a hydrolysis reaction is continuously performed, a tube type reactor is preferably used because it exhibits good operability and high resistance to pressure in a high pressure reaction. Furthermore, when a mixer is used, a static mixer is preferably used because it has a simple configuration and its maintenance can be performed in a simple manner. Specifically, an orifice contraction flow type mixer is more preferable because a fluid shear rate at a mixing section thereof is high and high mixing effect can be achieved.

After the completion of reaction, for example, a reacted mixture is cooled down to a desired temperature, evaporation or distillation, spontaneous sedimentation or centrifugal sedimentation is performed as desired according to a known method to refine the mixture and separate the mixture from unreacted water, thus obtaining a hydrolysate.

(Examples)

<Example 1> (Reference)

A reaction apparatus shown in FIG. **1** was used. The reaction apparatus of FIG. **1** includes a tube type reactor **1**, a cooler **2**, a raw material supply section **3**, a water supply section **4** and a separate collection tank **5**. Each of the raw material supply section **3** and the water supply section **4** is connected to the tube type reactor **1**.

After preheating to the same temperature as a reaction temperature, 2-ethyl-hexylglycidyl ether as a raw material and ion exchange water were continuously supplied at 0.73 g/min and 7.03 g/min, respectively, from the raw material supply section **3** and water supply section **4** to the tube type reactor **1** (having a flow channel minimum inner diameter of 1.0 mm, a tube length of 10 m and being formed of SUS316).

In the tube type reactor **1**, an inner fluid was heated so that a temperature (i.e., reaction temperature) of the inner fluid becomes 250 °C, a pressure (i.e., reaction pressure) in the tube type reactor **1** was controlled by a back pressure valve **6** so as to be 5MPa. Under the above-described temperature and pressure conditions, a hydrolysis reaction of an organic compound as a raw material was performed. The saturation vapor pressure of water at 250 °C was 4.0 MPa and the amount of water with respect to the organic compound as a raw material in a stationary state of the reaction was, in terms of molar conversion, 100 times as large as a stoichiometric amount of water required for the reaction.

After the hydrolysis reaction, a resultant mixture was cooled down to 40 °C to 50 °C in the cooler **2**, and then the mixture was collected in the separate collection tank **5** through the back pressure valve **6.** In the separate collection tank **5**, the reacted mixture was split into layers and a reactant as an upper layer was collected.

The reactant was sampled at one hour after glycidyl ether was supplied and the hydrolysis reaction was started, and a reaction conversion ratio and a dimer selective ratio for glyceryl ether were obtained from a gas chromatogram (a gas chromatography apparatus: Agilent 6850 Series II manufactured by Agilent Technologies, a capillary column: HP-ULTRA2 having dimensions of 12 m $\times$0.2 mm $\times$ 0.33 $\mu$m, an internal standard substance: n-decan). Results are shown in Table 1. The reaction conversion ratio was calculated based on: reacted glycidyl ether (mol) / supplied glycidyl ether (mol) $\times$ 100. The dimer production rate indicating a side reaction was calculated based on: a dimer amount (mol%) in the reactant / the reaction conversion ratio (mol%) $\times$ 100.

<Example 2> (Reference)

The tube type reactor **1** having a tube length of 3.1 m was used, the reaction temperature was adjusted to be 270 °C, the reaction pressure was adjusted to be 7 MPa, 2-ethyl-hexylglycidyl ether as a raw material was supplied at 0.23 g/min and ion exchange water was supplied at 2.2 g/min. Other than that, in the same manner as in Example 1, glyceryl ether was produced. Results are shown in Table 1. Note that a saturation vapor pressure of water at 270 °C is 5.5 MPa.

<Example 3> (Reference)

2-ethyl-hexylglycidyl ether as a raw material and ion exchange water were continuously supplied at 1.64 g/min and 15.9 g/min, respectively, to the tube type reactor **1** having a flow channel minimum inner diameter of 3.0 mm and

a tube length of 2.0 m. Other than that, in the same manner as in Example 2, glyceryl ether was produced. Results are shown in Table 1.

<Example 4> (Reference)

The reaction temperature was adjusted to be 290 °C and the reaction pressure was adjusted to be 9 MPa. Other than that, in the same manner as in Example 3, glyceryl ether was produced. Results are shown in Table 1. Note that a saturation vapor pressure of water at 290 °C is 7.4 MPa.

<Example 5> (Reference)

2-ethyl-hexylglycidyl ether as a raw material and ion exchange water were continuously supplied at 18.17 g/min and 176 g/min, respectively, to the tube type reactor **1** having a flow channel minimum inner diameter of 10 mm and a tube length of 2.0 m. Other than that, in the same manner as in Example 4, glyceryl ether was produced. Results are shown in Table 1.

<Example 6>

A reaction apparatus shown in FIG. **2** was used. The apparatus of FIG. **2** includes a tube type reactor **1**, a cooler **2**, a raw material supply section **3**, a water supply section **4**, a separate collection tank 5 and a mixer **7**. Each of the raw material supply section **3** and the water supply section **4** is connected to the mixer **7**.

After preheating to the same temperature as a reaction temperature, 2-ethyl-hexylglycidyl ether as a raw material and ion exchange water were continuously supplied at 67.9 g/min and 657 g/min, respectively, from the raw material supply section 3 and the water supply section **4** to the mixer **7**. A contraction flow type mixer (in this case, Bunsan-kun manufactured by Fujikin, including 5 pairs of 4-pore block and 5-pore block and having a contraction section inner diameter of 1.0 mmφ and a pore length of 0.05 m) was provided in the mixer **7**. Reaction materials mixed in the mixer **7** were continuously supplied to the tube type reactor **1** (having a flow channel inner diameter of 16 mm, a tube length of 5.4 m and being formed of SUS316).

In the tube type reactor **1**, an inner fluid was heated so that a temperature (reaction temperature) of the inner fluid become 270 °C and a pressure (i.e., reaction pressure) in the tube type reactor **1** was adjusted to be 7 MPa. Under the temperature and pressure conditions, a hydrolysis reaction of an organic compound as a raw material was performed. In a stationary state of a reaction, an amount of water with respect to the organic compound as a raw material was, in terms of molar conversion, 100 times as large as a stoichiometric amount of water required for a reaction.

After the hydrolysis reaction, a mixture was cooled down to 40 °C to 50 °C in the cooler **2**, and then the mixture was collected in the separate collection tank **5** through the back pressure valve **6**. In the separate collection tank **5**, the reacted mixture was split into layers and a reactant as an upper layer was collected.

The reactant was sampled at one hour after glycidyl ether was supplied and the hydrolysis reaction was started, and a reaction conversion ratio and a dimer selective ratio for glyceryl ether were obtained from the gas chromatogram. Results are shown in Table 1.

<Example 7>

The reaction temperature was adjusted to be 290 °C, the reaction pressure was adjusted to be 9 MPa and 2-ethyl-hexylglycidyl ether as a raw material and ion exchange water were continuously supplied at 102 g/min and 985 g/min, respectively, to the mixer **7**. Other than that, in the same manner as in Example 6, glyceryl ether was produced. Results are shown in Table 1.

<Example 8>

2-ethyl-hexylglycidyl ether as a raw material and ion exchange water were continuously supplied at 69.7 g/min and 472 g/min, respectively, to the mixer **7**. In a stationary state of a reaction, an amount of water with respect to the organic compound as a raw material was, in terms of molar conversion, 70 times as large as a stoichiometric amount of water required for a reaction. Other than that, as in the same manner as in Example 6, glyceryl ether was produced. Results are shown in Table 1.

<Example 9>

2-ethyl-hexylglycidyl ether as a raw material and ion exchange water were continuously supplied at 26.9 g/min

and 520 g/min, respectively, to the mixer **7**. In a stationary state of a reaction, an amount of water with respect to the organic compound as a raw material was, in terms of molar conversion, 200 times as large as a stoichiometric amount of water required for a reaction. Other than that, as in the same manner as in Example 8, glyceryl ether was produced. Results are shown in Table 1.

<Example 10>

The tube type reactor **1** having a tube length of 10.8 m was used, isodecylglycidyl ether as a raw material and ion exchange water were supplied at 77.0 g/min and 647 g/min, respectively, to the mixer **7**, the reaction temperature was adjusted to be 280 °C and the reaction pressure was adjusted to be 8 MPa. Other than that, in the same manner as in Example 6, glyceryl ether was produced. Results are shown in Table 1. Note that a saturation vapor pressure of water at 280 °C is 6.4 MPa.

<Example 11>

The reaction apparatus of FIG. **2** was used. After preheating to the same temperature as a reaction temperature, 2-ethyl-hexylglycidyl ether as a raw material and ion exchange water were continuously supplied at 4650 g/min and 45000 g/min, respectively, to the mixer **7**. A contraction flow type mixer (in this case, Bunsan-kun manufactured by Fujikin, including 5 pairs of 20-pore block and 25-pore block and having a contraction section inner diameter of 3.0 mmφ and a pore length of 0.05 m) was provided in the mixer **7**. Reaction materials mixed in the mixer **7** were continuously supplied to the tube type reactor **1** (having a flow channel inner diameter of 50 mm and a tube length of 74 m).

In the tube type reactor **1**, an inner fluid was heated so that the reaction temperature become 250 °C and the reaction pressure was adjusted to be 8 MPa. Under the temperature and pressure conditions, a hydrolysis reaction of an organic compound as a raw material was performed. Results are shown in Table 1.

<Comparative Example 1>

Instead of the reaction apparatus of FIG. **2**, the reaction apparatus of FIG. **1** was used. That is, the mixer **7** was not used. Other than that, in the same manner as in Example 6, glyceryl ether was produced. Results are shown in Table 1.

<Comparative Example 2>

Instead of the reaction apparatus of FIG. **2**, the reaction apparatus of FIG. **1** was used. That is, the mixer **7** was not used. Other than that, in the same manner as in Example 8, glyceryl ether was produced. Results are shown in Table 1.

<Comparative Example 3>

Instead of the reaction apparatus of FIG. **2**, the reaction apparatus of FIG. **1** was used. That is, the mixer **7** was not used. Other than that, in the same manner as in Example 9, glyceryl ether was produced. Results are shown in Table 1.

<Comparative Example 4>

Instead of the reaction apparatus of FIG. **2**, the reaction apparatus of FIG. **1** was used. That is, the mixer **7** was not used. Other than that, in the same manner as in Example 10, glyceryl ether was produced. Results are shown in Table 1.

In each of Examples 1 through 5 and Comparative Examples 1 through 4, a reactive apparatus which does not include the mixer **7** was used. However, the reactor **1** serves as a mixing section and a reaction section.

[Table 1]

| | | Example 1* | Example 2* | Example 3* | Example 4* | Example 5* | Example 6 | Example 7 | Example 8 | Example 9 | Example 10 | Example 11 | Comparative Example 1 | Comparative Example 2 | Comparative Example 3 | Comparative Example 4 |
|---|---|---|---|---|---|---|---|---|---|---|---|---|---|---|---|---|
| Organic compound as raw material * | | A | A | A | A | A | A | A | A | A | B | A | A | A | A | B |
| Flow rate of reaction materials | | | | | | | | | | | | | | | | |
| Organic copound as raw material | (g/min) | 0.73 | 0.23 | 1.64 | 1.64 | 18.17 | 67.9 | 102 | 69.7 | 26.9 | 77.0 | 4,650 | 67.9 | 69.7 | 26.9 | 77.0 |
| | (ml/min) | 1.02 | 0.33 | 2.35 | 2.41 | 26.70 | 97.45 | 150 | 100 | 38.6 | 111 | 6,522 | 97.4 | 100 | 38.6 | 111 |
| Water | (g/min) | 7.03 | 2.20 | 15.9 | 15.9 | 176 | 657 | 985 | 472 | 520 | 647 | 45,000 | 657 | 472 | 520 | 647 |
| | (ml/min) | 9.17 | 3.01 | 21.7 | 22.9 | 254 | 898 | 1,420 | 645 | 711 | 907 | 58,693 | 898 | 645 | 711 | 907 |
| Mixer | | — | — | — | — | — | Contraction flow type | Contraction flow type | Contraction flow type | Contraction flow type | Contraction flow type | Contraction flow type | — | — | — | — |
| Flow channel minimum inner diameter Dmin | (mm) | — | — | — | — | — | 1.0 | 1.0 | 1.0 | 1.0 | 1.0 | 3.0 | — | — | — | — |
| Tube length | (m) | — | — | — | — | — | 0.05 | 0.05 | 0.05 | 0.05 | 0.05 | 0.05 | — | — | — | — |
| Residence time t | (min) | — | — | — | — | — | 0.11 | 0.071 | 0.14 | 0.14 | 0.11 | 0.013 | — | — | — | — |
| Reactor | | Tube type | Tube type | Tube type | Tube type | Tube type | Tube type | Tube type | Tube type | Tube type | Tube type | Tube type | Tube type | Tube type | Tube type | Tube type |
| Flow channel minimum inner diameter Dmin | (mm) | 1.0 | 1.0 | 3.0 | 3.0 | 10 | 16 | 16 | 16 | 16 | 16 | 50 | 16 | 16 | 16 | 16 |
| Tube length | (m) | 10 | 3.1 | 2.0 | 2.0 | 2.0 | 5.4 | 5.4 | 5.4 | 5.4 | 10.8 | 74 | 5.4 | 5.4 | 5.4 | 10.8 |
| Residence time t | (min) | 1.0 | 1.0 | 0.8 | 0.8 | 0.8 | 1.5 | 1.0 | 2.0 | 2.0 | 3.0 | 3.0 | 1.5 | 2.0 | 2.0 | 3.0 |
| Mixer or reactor | | | | | | | | | | | | | | | | |
| Flow channel minimum inner diameter Dmin | (mm) | 1.0 | 1.0 | 3.0 | 3.0 | 10 | 1.0 | 1.0 | 1.0 | 1.0 | 1.0 | 3.0 | 16 | 16 | 16 | 16 |
| The number n of flow channel minimum inner diameter | (-) | 1 | 1 | 1 | 1 | 1 | 4 | 4 | 4 | 4 | 4 | 20 | 1 | 1 | 1 | 1 |
| Flow rate U | (mm/sec) | 216 | 71 | 57 | 60 | 59 | 5,280 | 8,326 | 3,951 | 3,974 | 5,405 | 7,689 | 83 | 63 | 64 | 83 |
| U/Dmin | (1/sec) | 216 | 71 | 19 | 20 | 5.9 | 5,280 | 8,326 | 3,951 | 3,974 | 5,405 | 2,563 | 5.2 | 4.0 | 4.0 | 5.2 |
| Reaction conditions | | | | | | | | | | | | | | | | |
| Reaction temperature | (°C) | 250 | 270 | 270 | 290 | 290 | 270 | 290 | 270 | 270 | 280 | 250 | 270 | 270 | 270 | 280 |
| Reaction pressure | (MPa) | 5 | 7 | 7 | 9 | 9 | 7 | 9 | 7 | 7 | 8 | 8 | 7 | 7 | 7 | 8 |
| Mixing ratio of water/organic compound as raw material (molar ratio) | (-) | 100 | 100 | 100 | 100 | 100 | 100 | 100 | 70 | 200 | 100 | 100 | 100 | 70 | 200 | 100 |
| Reaction results | | | | | | | | | | | | | | | | |
| Conversion ratio | (mol%) | 99.5 | 99.2 | 97.8 | 100.0 | 99.3 | 82.8 | 100.0 | 83.7 | 99.1 | 96.5 | 99.6 | 60.3 | 66.8 | 76.7 | 94.9 |
| Dimer amount in reactant | (mol%) | 1.4 | 1.2 | 2.0 | 2.2 | 2.1 | 1.8 | 1.6 | 2.1 | 1.7 | 3.8 | 3.2 | 1.2 | 1.7 | 1.2 | 5.1 |
| Dimer selective ratio | (mol%) | 1.4 | 1.3 | 2.1 | 2.2 | 2.1 | 2.1 | 1.6 | 2.5 | 1.7 | 3.9 | 3.2 | 1.9 | 2.6 | 1.6 | 5.4 |

* Organic compound as raw material A: 2-ethyl-hexylglycidyl ether
Organic compound as raw material B: isodecylglycidyl ether

*Examples 1 to 5 are Reference Examples

According to the results described above, compared to the comparative examples, in each of the Examples,

the conversion ratio is high and the dimer selective ratio is low. This shows that a hydrolysis reaction was efficiently performed.

INDUSTRIAL APPLICABILITY

As has been described, the present invention is useful as a method for producing a hydrolysate , in which an organic compound and water are mixed and a hydrolysis reaction of the organic compound is performed. A hydrolysate obtained according to the present invention, e.g., glyceryl ether obtained by hydrolysis of glycidyl ether can be used as a solvent, an emulsifier, a dispersant, a detergent, or a foam booster.

## Claims

1. A method for producing a hydrolysate in which an organic compound and water are mixed by flowing the organic compound and water through an orifice contraction flow type mixer and a hydrolysis reaction of the organic compound is performed,
   wherein the mixing is performed under shear flow of the organic compound and the water at a shear rate U/Dmin of 20 $sec^{-1}$ or more, where Dmin is a flow channel minimum inner diameter (mm) in a mixing section and U is a flow rate (mm/sec) of a mixture of the organic compound and the water in the mixing section,
   the hydrolysis reaction of the organic compound is performed at a reaction temperature of 150°C to 350°C and a reaction pressure equal to or higher than a saturation vapor pressure of the water,
   wherein after mixing the organic compound and the water, the hydrolysis reaction of the organic compound is subsequently performed, and
   wherein the organic compound is glycidyl ether expressed by a general formula (I)

   [Chemical formula 1]

   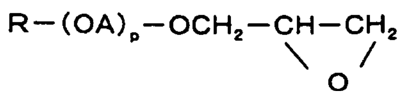

   (I)

   (where R is a hydrocarbon group in which part or all hydrogen atoms may be replaced with fluorine atoms, of which carbon number is 1 to 20 and which is saturated or unsaturated, OA is an oxyalkylene group which may be the same as or different from another OA and of which carbon number is 2 to 4, and p is a number of 0 to 20).

2. The method of claim 1, wherein the hydrolysis reaction of the organic compound is performed without using a catalyst.

3. The method of claim 1, wherein the hydrolysis reaction of the organic compound is a ring-opening reaction by the water.

4. The method of claim 1, wherein an amount of the water with respect to the organic compound is, in terms of molar conversion, 20 to 500 times as large as a stoichiometric amount of water required for the reaction.

5. The method of claim 1, wherein the hydrolysis reaction of the organic compound is continuously performed.

6. The method of claim 1, wherein the flow channel minimum inner diameter in the mixing section in which the organic compound and the water are mixed is 1 mm to 15 mm.

7. The method of claim 1, wherein the reaction temperature for the hydrolysis reaction ranges from 250 to 290°C.

## Patentansprüche

1. Verfahren zur Erzeugung eines Hydrolysates, worin eine organische Verbindung und Wasser gemischt werden, durch Fließen der organischen Verbindung und Wasser durch einen Mischer vom Öffnungskonzentrationsflusstyp und wobei eine Hydrolysereaktion der organischen Verbindung durchgeführt wird:

   worin das Mischen unter einem Scherfluss der organischen Verbindung und Wasser bei einer Scherrate U/Dmin

von 20 $s^{-1}$ oder mehr durchgeführt wird, worin Dmin ein minimaler Innendurchmesser des Fließkanals (mm) in einem Mischbereich ist und U eine Fließrate (mm/s) einer Mischung aus der organischen Verbindung und Wasser in dem Mischbereich ist,
worin die Hydrolysereaktion der organischen Verbindung bei einer Reaktionstemperatur von 150°C bis 350°C und einem Reaktionsdruck durchgeführt wird, der gleich oder höher ist als ein Sättigungsdampfdruck von Wasser,
worin nach Mischen der organischen Verbindung und Wasser die Hydrolysereaktion der organischen Verbindung anschließend durchgeführt wird und worin die organische Verbindung Glycidylether mit der allgemeinen Formel (I) ist

[Chemische Formel 1]

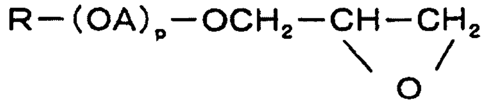
(I)

(worin R eine Kohlenwasserstoffgruppe ist, worin ein Teil oder alle Wasserstoffatome durch Fluoratome ersetzt sein können, wobei die Kohlenstoffzahl 1 bis 20 ist und die gesättigt oder ungesättigt ist, OA eine Oxyalkylengruppe ist, die gleich oder verschieden von einem anderen OA sein kann und wobei die Kohlenstoffzahl 2 bis 4 ist und p eine Zahl von 0 bis 20 ist).

**2.** Verfahren nach Anspruch 1, worin die Hydrolysereaktion der organischen Verbindung ohne Verwendung eines Katalysators durchgeführt wird.

**3.** Verfahren nach Anspruch 1, worin die Hydrolysereaktion der organischen Verbindung eine Ringöffnungsreaktion durch das Wasser ist.

**4.** Verfahren nach Anspruch 1, worin eine Menge an Wasser in Bezug auf die organische Verbindung, ausgedrückt als molare Umwandlung, das 20- bis 500-fache in Bezug auf die stöchiometrische Menge an Wasser ist, das für die Reaktion erforderlich ist.

**5.** Verfahren nach Anspruch 1, worin die Hydrolysereaktion der organischen Verbindung kontinuierlich durchgeführt wird.

**6.** Verfahren nach Anspruch 1, worin der minimale Innendurchmesser des Fließkanals in dem Mischbereich, in dem die organische Verbindung und das Wasser gemischt werden, 1 bis 15 mm ist.

**7.** Verfahren nach Anspruch 1, worin die Reaktionstemperatur für die Hydrolysereaktion im Bereich von 250°C bis 290°C liegt.

## Revendications

**1.** Procédé de production d'un hydrolysat dans lequel un composé organique et de l'eau sont mélangés en faisant circuler le composé organique et l'eau à travers un mélangeur de type à écoulement par contraction de flux et une réaction d'hydrolyse du composé organique est réalisée,
dans lequel le mélange est réalisé sous un écoulement de cisaillement du composé organique et de l'eau à une vitesse de cisaillement U/Dmin d'au moins 20 $s^{-1}$, où Dmin est un diamètre interne minimum du canal d'écoulement (mm) dans une section de mélange et U est une vitesse d'écoulement (mm/s) d'un mélange du composé organique et de l'eau dans la section de mélange,
la réaction d'hydrolyse du composé organique est réalisée à une température de réaction comprise entre 150 °C et 350 °C et à une pression de réaction égale ou supérieure à une pression de vapeur saturante de l'eau,
dans lequel après le mélange du composé organique et de l'eau, la réaction d'hydrolyse du composé organique est ensuite réalisée, et
dans lequel le composé organique est de l'éther de glycidyle exprimé par une formule générale (I)

[Formule Chimique 1]

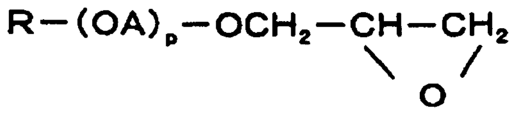

(I)

(où R représente un groupe hydrocarboné dans lequel une partie ou tous les atomes d'hydrogène peuvent être remplacés par des atomes de fluor, le nombre de carbone étant compris entre 1 et 20 et qui est saturé ou non saturé, OA représente un groupe oxyalkylène qui peut être identique ou différent d'un autre OA, le nombre de carbone étant compris entre 2 et 4, et p est un nombre compris entre 0 et 20).

**2.** Procédé selon la revendication 1, dans lequel la réaction d'hydrolyse du composé organique est réalisée sans utiliser un catalyseur.

**3.** Procédé selon la revendication 1, dans lequel la réaction d'hydrolyse du composé organique est une réaction d'ouverture de cycle par de l'eau.

**4.** Procédé selon la revendication 1, dans lequel une quantité d'eau par rapport au composé organique est, en termes de conversion molaire, de 20 à 500 fois plus importante qu'une quantité stoechiométrique d'eau nécessaire pour la réaction.

**5.** Procédé selon la revendication 1, dans lequel la réaction d'hydrolyse du composé organique est réalisée de façon continue.

**6.** Procédé selon la revendication 1, dans lequel le diamètre interne minimum du canal d'écoulement dans la section de mélange dans laquelle le composé organique et l'eau sont mélangés est compris entre 1 mm et 15 mm.

**7.** Procédé selon la revendication 1, dans lequel la température de réaction pour la réaction d'hydrolyse est comprise entre 250 °C et 290 °C.

FIG.1

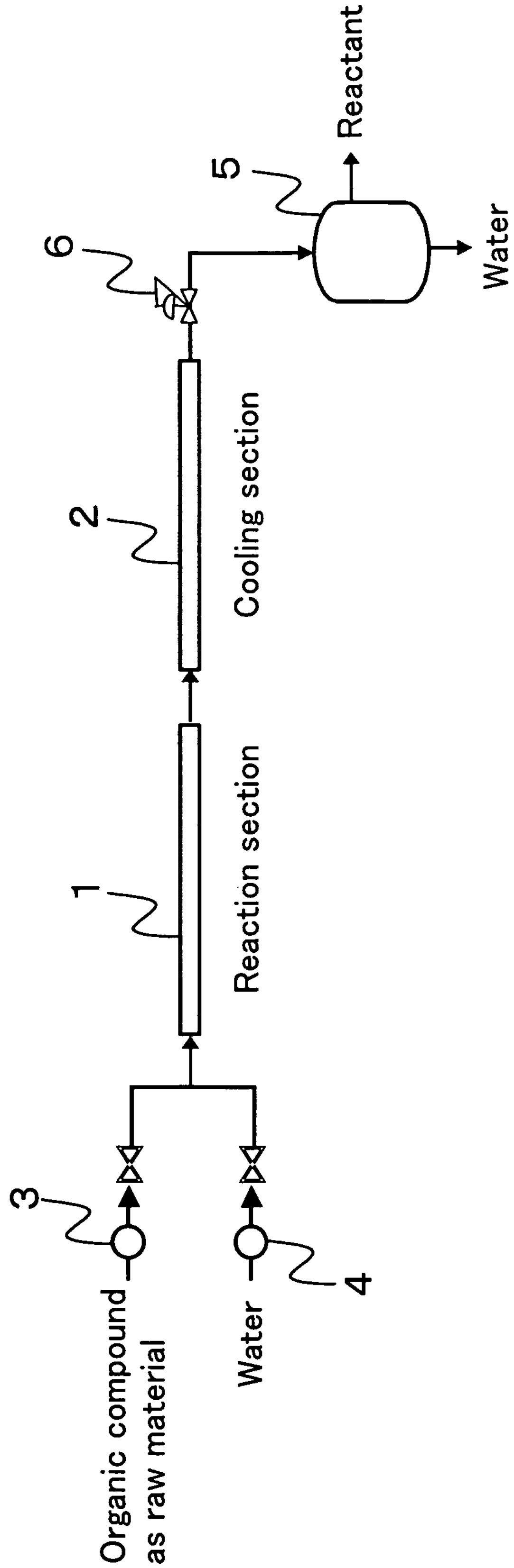
Organic compound as raw material
Water
3
4
1
Reaction section
2
Cooling section
6
5
Reactant
Water

FIG.2

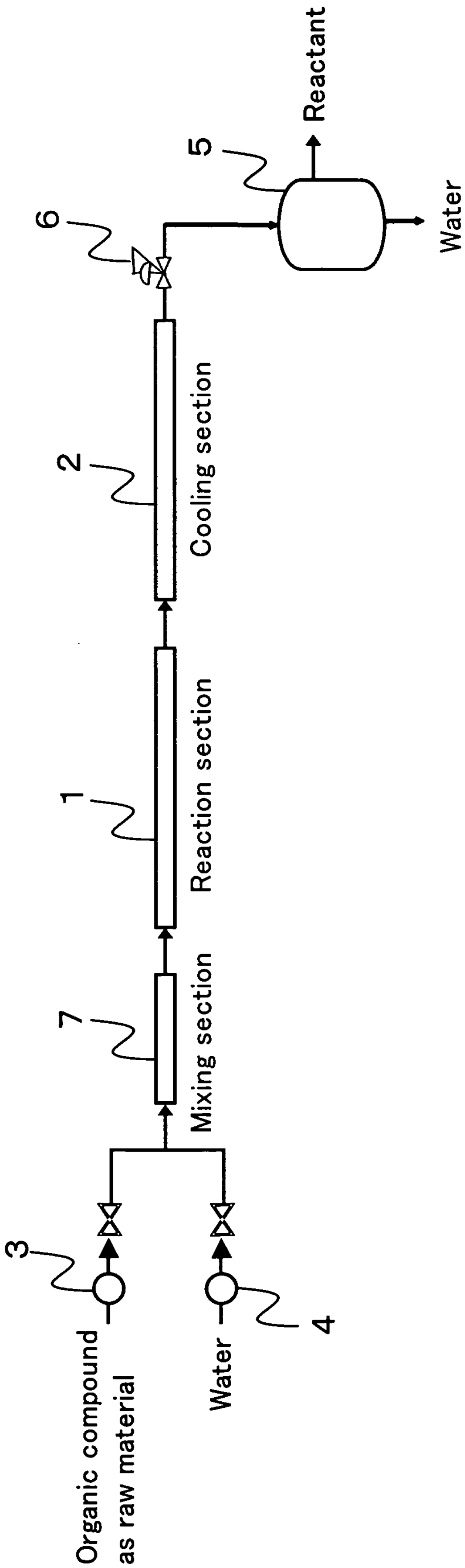
Organic compound as raw material
Water
3
4
7
Mixing section
1
Reaction section
2
Cooling section
6
5
Reactant
Water

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2002088000 A
- JP 2003267920 A